# EUROPEAN PATENT APPLICATION

(11) **EP 4 316 601 A1**
(43) Date of publication of application: **07.02.2024**
(21) Application number: 22781207.0
(22) Date of filing: 31.03.2022
(51) Int. Cl.: A61Q 5/00, A61Q 5/02, A61Q 5/04, A61Q 5/06, A61Q 5/08, A61Q 5/10, A61Q 5/12, A61K 8/37, A61K 8/46

(54) **HAIR TREATMENT COMPOSITION AND HAIR TREATMENT METHOD**

(30) Priority: 31.03.2021 JP 2021059023
(71) Applicant: B. Pro Co., Ltd., Kobe-shi, Hyogo 657-0841 (JP); Biotop Maya Co., Ltd., Sakai-shi, Osaka 590-0005 (JP); SO Pharma Inc., Sakai-shi,Osaka 590-0153 (JP); Diana Co., Ltd., Tokyo 151-0063 (JP)
(72) Inventor: TANAKA Yoshinobu, Kobe-shi, Hyogo 658-0021 (JP); TSUJINO Yoshio, Kobe-shi, Hyogo 658-0003 (JP)
(74) Representative: Michalski Hüttermann & Partner Patentanwälte mbB
(86) International application number: PCT/JP2022/016376
(87) International publication number: WO 2022/210997

(57) **Abstract**

[Object] There has been a demand for a hair treatment composition that has a long-lasting effect and ensures safety for the purpose of improving hair quality in hair straightening, dyeing, and bleaching, improving hair quality in a wave perm and a straight perm, shampooing, and hair treatment.

[Solution] The present invention provides a hair treatment composition that contains, as an active ingredient, a complex composed of a dicarboxylic acid and a thiol, in which the dicarboxylic acid is one or more selected from adipic acid, muconic acid, and cosmetically acceptable salts thereof. Further provided is a method for treating hair using the hair treatment composition.

## Description

### FIELD OF INVENTION

The present invention relates to a hair treatment composition that contains, as an active ingredient, a complex composed of a difunctional compound, preferably a difunctional compound having 6 or more carbon atoms (C6), and a thiol, and also relates to a method for treating hair using the hair treatment composition.

### BACKGROUND ART

Many hair treatment compositions containing organic acids, such as difunctional compounds, have been developed in order to improve the condition of hair. Disclosed examples thereof include a perming agent containing thioglycolic acid, adipic acid, and so forth (see Patent Literature 1); a primary hair shape-controlling agent composition containing a hair-reducing substance having a thiol group and a difunctional compound, preferably adipic acid, (see Patent Literature 2); and a hair treatment method in which a first composition containing a difunctional compound, such as adipic acid or muconic acid, and a second composition having a different pH are applied to hair, and in which in an embodiment, the composition does not contain a reducing agent (see Patent Literature 3).

### CITATION LIST

### PATENT LITERATURE

PTL 1: International Publication No. 2006/011771
PTL 2: Japanese Patent No. 5112836
PTL 3: International Publication No. 2018/191362

### SUMMARY OF THE INVENTION

### TECHNICAL PROBLEM

There has been a demand for a hair treatment composition that has a long-lasting effect and ensures safety for the purpose of improving hair quality in hair straightening, dyeing, and bleaching, improving hair quality in a wave perm and a straight perm, shampooing, and hair treatment. In each of Patent Literatures 1 and 2, a primary perming agent containing a dicarboxylic acid is disclosed. The object thereof is to suppress damage due to excessive shrinkage of hair during the reduction reaction of the hair by the primary perming agent, and the problem to be solved by the present invention is different from the problem to be solved by those inventions. Patent Literature 3 discloses a composition that does not contain a reducing agent in an embodiment, and the dicarboxylic acid acts on hair alone.

### SOLUTION TO PROBLEM

The present invention has been accomplished by the inventors through the finding that a complex composed of a specific dicarboxylic acid and a thiol provides a marked improvement effect on hair. That is, according to the present invention, a hair treatment composition contains, as an active ingredient, a complex composed of a dicarboxylic acid and a thiol, in which the dicarboxylic acid is one or more selected from the group consisting of dicarboxylic acids each having 6 to 10 carbon atoms and cosmetically acceptable salts thereof, and the thiol is one or more selected from the group consisting of aminothiols and cosmetically acceptable salts thereof. The complex may be a complex formed by a reaction at a mole ratio of thiol/dicarboxylic acid = 0.01 to 100. The composition may be a complex obtained by a reaction at a mole ratio of thiol/dicarboxylic acid = 1 or more.

According to another aspect of the present invention, a hair treatment composition contains a dicarboxylic acid and a thiol, in which the dicarboxylic acid is one or more selected from the group consisting of dicarboxylic acids each having 6 to 10 carbon atoms and cosmetically acceptable salts thereof, and the thiol is one or more selected from the group consisting of aminothiols and cosmetically acceptable salts thereof.

Furthermore, according to the present invention, the hair treatment composition contains substantially no active ingredient other than the dicarboxylic acid, the thiol, and the complex composed of the dicarboxylic acid and the thiol. Another aspect of the present invention provides one or more selected from the group consisting of a hair straightening agent, a hair quality improver for use in dyeing or bleaching, a hair quality improver for use in a wave perm or a straight perm, a shampoo agent, a hair treatment agent, and a hair styling agent (hair dressing). Another aspect of the present invention does not provide a primary agent or a secondary agent for a wave perm or a straight perm.

The thiol in the present invention is an aminothiol and specifically, may be one or more selected from the group consisting of cysteamine, cysteine, amidinocysteine, aminothiophenol, mercaptopyridine, homocysteine, aminoundecanethiol, and cosmetically acceptable salts thereof. The dicarboxylic acid in the present invention may be one or more selected from the group consisting of adipic acid, pimelic acid, suberic acid, azelaic acid, hexenedioic acid, heptenedioic acid, octenedioic acid, muconic acid, and cosmetically acceptable salts thereof.

The hair treatment composition of the present invention is obtained by a production method including at least a mixing step of mixing the dicarboxylic acid and the thiol at a high temperature. The complex formed of the dicarboxylic acid and the thiol is obtained by the mixing step. The mixing step may be a mixing step of mixing the dicarboxylic acid with the thiol at 60°C to 100°C. The mixing step may be a mixing step of performing mixing in an environment where an active ingredient other than the dicarboxylic acid, the thiol, a solvent therefor, and a pH adjuster is substantially absent.

According to the present invention, a method for treating hair includes at least an application step of applying the above-described hair treatment composition or a hair treatment composition produced by the above-described production method to hair, and after the application step, a heating step of heating the hair. The heating step may be a step of heating the hair in such a manner that the surface temperature of the hair is 40°C to 60°C, 80°C, 100°C, 120°C, 140°C, 160°C, or 180°C.

Another method for treating hair of the present invention excludes a method for applying the hair treatment composition to hair as a primary agent or a secondary agent for a wave perm or a straight perm, and is intended for one or more selected from the group consisting of hair straightening, improvements in hair quality during dyeing and bleaching, improvements in hair quality during a wave perm and a straight perm, shampooing, and hair treatment. The method for treating hair of the present invention may be a non-therapeutic method.

### ADVANTAGEOUS EFFECTS OF INVENTION

The present invention can provide the hair treatment composition, which is composed of a dicarboxylic acid and a thiol that have been conventionally used as cosmetic materials, and thus ensures safety for users. In the method for treating hair using the hair treatment composition of the present invention, hair straightening, improvements in hair quality during dyeing and bleaching, improvements in hair quality during a wave perm and a straight perm, shampooing, and hair treatment can be performed, and the effects are maintained even after multiple shampoo washes.

When the hair treatment composition of the present invention is used in hair straightening, the finished hair is soft and free of twisting. Even after the hair straightening treatment, the hair straightening effect on the hair and the strong and soft state of the hair are maintained. When the hair treatment composition of the present invention is used in dyeing and bleaching, the finished hair can be soft with high elasticity. Damage to the hair during bleaching is reduced, and twisting of the hair is reduced. When the hair treatment composition of the present invention is used in a wave perm and a straight perm, the finished hair can be soft, and the effect of the perm on the hair and the strong and soft state are maintained. When the hair treatment composition of the present invention is used in shampooing and hair treatment, unintended waviness of the finished hair can be relieved, the elasticity of the finished hair can be increased, and the finished hair can be soft. These effects can persist even after multiple washes of the hair with a shampoo.

In particular, in the method for treating hair of the present invention, the effect is obtained by heating the hair after applying the hair treatment composition to the hair, and it is advantageous in that the effect is obtained even if the heating step is a step of setting the surface temperature of the hair to 40°C to 60°C. This temperature range is a temperature range for daily hairdressing at home, and the risk of damaging the hair is low. Thus, the composition and method of the present invention can provide hair treatment effects without using specialized equipment.

### BRIEF DESCRIPTION OF THE DRAWINGS

[Fig. 1] Fig. 1 is a photograph illustrating the effects of improving the curl formation and the persistence of hair treated with the hair treatment composition of the present invention as an intermediate treatment agent of a permanent wave agent.
[Fig. 2] Fig. 2 is a photograph illustrating the straightening effect of a hair treatment composition of the present invention on the frizzy hair of an Ethiopian woman.
[Fig. 3] Fig. 3 is a photograph illustrating the effects of improving the curl formation and persistence of bleached hair treated with a hair treatment composition of the present invention.
[Fig. 4] Fig. 4 is a photograph illustrating the curl formation of bleached hair treated with a shampoo agent containing a hair treatment composition of the present invention. Description of Embodiments

While the present invention will be described in detail on the basis of embodiments thereof, the present invention is not limited to the embodiments.

A hair treatment composition according to the present invention contains, as an active ingredient, a complex composed of a difunctional compound, preferably a difunctional compound having 6 or more carbon atoms (C6), and a thiol. The difunctional compound is a general term for compounds containing two functional groups, and specific examples thereof include dicarboxylic acids, which are dicarbonyl compounds. In the hair treatment composition of the present invention, the complex composed of a dicarboxylic acid and a thiol contained in the composition acts on hair as an active ingredient. In the present invention, the complex refers to a state in which multiple molecules are associated or bound by an intermolecular force or a covalent bond, and includes a compound or an associate composed of multiple molecules. In the hair treatment composition of the present invention, a complex formed by the reaction of a thiol and a dicarboxylic acid acts as an active ingredient.

In one embodiment of the present invention, a hair treatment composition contains a dicarboxylic acid and a thiol. The complex formed of the dicarboxylic acid and the thiol acts on hair as an active ingredient, but the dicarboxylic acid and/or the thiol may act on hair alone. Specifically, the hair treatment composition of the present invention may contain a dicarboxylic acid and a thiol, in which the dicarboxylic acid may be one or more selected from the group consisting of muconic acid, adipic acid, octenedioic acid, and cosmetically acceptable salts thereof. The thiol may be one or more selected from the group consisting of aminothiols and cosmetically acceptable salts thereof, and may be cysteamine or a cosmetically acceptable salt thereof.

The complex, which is an active ingredient of the present invention, is a complex that can be formed by reacting a dicarboxylic acid and a thiol in a solvent, such as water or ethanol, preferably in an aqueous solution. Specific examples thereof include a complex induced by a thiol-ene reaction in which a thiol group contained in a thiol interacts with a double bond of an alkene contained in a dicarboxylic acid or an alkene formed by reduction of a dicarboxylic acid; and a complex induced by an intermolecular hydrogen bond formed between a dicarboxylic acid and a thiol. The complex can be obtained by a mixing step in which a dicarboxylic acid and a thiol are mixed at a high temperature.

A dicarboxylic acid refers to a compound having two carboxy groups. In the hair treatment composition of the present invention, α,ω-dicarboxylic acid is preferably used among dicarboxylic acids. A linear or branched alkane or alkene having 6 to 10 carbon atoms with carboxy groups added to both ends is used. More preferably, a linear or branched alkane or alkene having 6 to 8 carbon atoms with carboxy groups added to both ends is used.

As the dicarboxylic acid used in the hair treatment composition of the present invention, a linear saturated dicarboxylic acid and/or unsaturated dicarboxylic acid is preferably used. Examples of the saturated dicarboxylic acid include hexanedioic acid, heptanedioic acid, octanedioic acid, nonanedioic acid, and decanedioic acid. Examples of the unsaturated dicarboxylic acid include hexenedioic acid, heptenedioic acid, octenedioic acid, nonenedioic acid, decenedioic acid, hexadienedioic acid, heptadienoic acid, octadienedioic acid, nonadienedioic acid, and decadienedioic acid.

The dicarboxylic acid used in the hair treatment composition of the present invention may be one or more selected from adipic acid, pimelic acid, suberic acid, azelaic acid, hexenedioic acid, heptenedioic acid, octenedioic acid, muconic acid, and cosmetically acceptable salts thereof, more preferably one or more selected from the group consisting of adipic acid, octenedioic acid, muconic acid, and cosmetically acceptable salts thereof. A high hair-treating effect has been provided in the hair treatment compositions containing the complex of the dicarboxylic acid and the thiol.

With respect to a dicarboxylic acid in which cis-trans isomers are present, any isomer can be used as long as the effects of the present invention is provided. For example, there are three cis-trans isomers of muconic acid: trans-trans conformation; cis-trans conformation; and cis-cis conformation. When muconic acid is used as the dicarboxylic acid, any isomer can be used in the present invention. However, trans-trans muconic acid can advantageously form a complex with a thiol in a short time, compared with other isomers.

A thiol refers to a compound having at least one thiol group. The thiol used in the hair treatment composition together with the dicarboxylic acid in the present invention is not limited as long as the effects of the present invention are provided. Specifically, the thiol is an aminothiol. The aminothiol, also referred to as mercaptoamine, is a thiol having one or more amino groups.

Specifically, the aminothiol used in the hair treatment composition of the present invention may be one or more selected from the group consisting of cysteamine, cysteine, amidinocysteine, aminothiophenol, mercaptopyridine, homocysteine, aminoundecanethiol, and cosmetically acceptable salts thereof. Among these thiols, a compound having a primary amine, such as cysteamine or cysteine, is preferably used, and cysteamine is more preferably used.

The pH of the hair treatment composition of the present invention is preferably slightly acidic to slightly alkaline, more preferably in the neutral range. Specifically, the pH is 5 to 8, more preferably 5.5 to 7.5, still more preferably 6 to 7. After the mixing step described below, the pH of the composition can be adjusted to a desired pH.

The hair composition of the present invention can also be incorporated into a hair dye, a bleaching agent, and a perming agent. In this case, the pH of the hair treatment composition of the present invention is preferably neutral to alkaline, more preferably neutral to slightly alkaline. Specifically, the pH is 7 to 12, preferably 8 to 11.

The hair treatment composition of the present invention contains the complex formed of the dicarboxylic acid and the thiol as an active ingredient, and may or need not contain an active ingredient other than the dicarboxylic acid, the thiol, and the complex thereof. This is because even if the composition does not contain an active ingredient other than the dicarboxylic acid, the thiols, and the complex thereof, the composition containing at least the complex can provide an effect on hair. The active ingredient other than the dicarboxylic acid, the thiol, and the complex thereof refers to an ingredient that imparts some effect to hair when applied to the hair. Specific examples thereof include reducing agents other than thiols, e.g., disulfide compounds and sodium sulfite; oxidizing agents, such as sodium bromate and an aqueous hydrogen peroxide solution; humectants, such as hyaluronic acid, chondroitin sulfate, collagen, glycerol, xylitol, glucose, sucrose, amino acids, derivatives thereof, and salts thereof; thickeners, such as xanthan gum, guar gum, and pectin; and surfactants, such as anionic surfactants, cationic surfactants, amphoteric surfactants, and nonionic surfactants. Here, the active ingredient other than the dicarboxylic acid, the thiol, and the complex thereof do not include an ingredient that is not intended to directly provide an effect on hair, such as an antioxidant, an antiseptic, a preservative, a fragrance, a colorant, and a pH adjuster.

In another embodiment of the present invention, a hair treatment composition is obtained by a method including at least a mixing step of mixing a dicarboxylic acid and a thiol at a high temperature. This is because the mixing at a high temperature promotes the formation of a complex, serving as an active ingredient, of the dicarboxylic acid and the thiol.

In the mixing step, in addition to the dicarboxylic acid and the thiol, the presence of a solvent (such as water or ethanol) and a pH adjuster that do not inhibit the formation of a complex of the dicarboxylic acid and the thiol is allowed. However, it is preferable to mix the dicarboxylic acid and the thiol in an environment where an active ingredient other than those is substantially absent. This is because mixing in the presence of other ingredients may inhibit the formation of a complex of the dicarboxylic acid and the thiol.

Specifically, the mixing step is a step of mixing the dicarboxylic acid and the thiol dissolved in a solvent (such as water or ethanol) in a high-temperature environment. Desirably, an ingredient that inhibits the formation of the complex of the dicarboxylic acid and the thiol is substantially absent in the solvent (such as water or ethanol), except for an acid or an alkali for pH adjustment. The term "substantially absent" means that an ingredient is not intentionally contained in view of the production process of the hair treatment composition, and the presence of a substance originally dissolved in the solvent (such as water or ethanol) or a substance dissolved in a solvent from the ambient atmosphere during the production process is allowed.

In one embodiment of the present invention, the mixing step is a production method in which mixing is performed in an environment where an active ingredient other than the dicarboxylic acid, the thiol, a solvent therefor (such as water or ethanol), and the pH adjuster is substantially absent. Here, the active ingredient that is not allowed to be present may inhibit the formation of the complex of the dicarboxylic acid and the thiol. The presence of an ingredient that does not inhibit the formation of the complex of the dicarboxylic acid and the thiol is allowed.

In the mixing step, the temperature at which the dicarboxylic acid and the thiol are mixed is 40°C to 120°C, preferably 60°C to 100°C. The temperature is more preferably 80°C to 100°C. When the solvent is water, the solvent may be heated to a boiling state at normal atmospheric pressure. The time for holding the mixture at a high temperature is not limited, but is specifically 1 minute to 24 hours, preferably 1 hour to 6 hours. The mixing step may be performed under high pressure. In such a case, the complex can be obtained even at a lower temperature or for a shorter time as compared with the mixing step under normal pressure.

In the mixing step, the pH adjustment of the solvent for mixing the dicarboxylic acid with the thiol is not essential. When the dicarboxylic acid and/or the thiol is insufficiently dissolved in the solvent, the pH adjustment promotes the dissolution. The dicarboxylic acid is acidic; hence, the mixing may be performed in the presence of an alkaline pH adjuster. The pH of the solvent at the time of mixing is not limited, but is preferably from an acidic region to a slightly alkaline region, more preferably in an acidic region. Specifically, the pH may be 4 to 9, 4 to 7, or 4 to 6.

The amounts-of-substance of dicarboxylic acid and thiol (mole ratio) in the hair treatment composition of the present invention are not limited, and the mole ratio may be thiol/dicarboxylic acid = 0.01 to 100 (that is, the mole ratio of the amount-of-substance of thiol to the amount-of-substance of dicarboxylic acid may be 0.01 to 100). The mole ratio is preferably thiol/dicarboxylic acid = 1 or more (that is, the mole ratio of the amount-of-substance of thiol to the amount-of-substance of dicarboxylic acid is 1 or more). Specifically, the ratio of thiol/dicarboxylic acid may be 1 to 100, 1 to 10, 1 to 5, or 1 to 3. This is because when the amount-of-substance of thiol is less than the amount-of-substance of dicarboxylic acid, the formation of the complex may be insufficient to fail to sufficiently provide the effect of improving hair quality.

The present invention provides a method for treating hair including at least an application step of applying a hair treatment composition to hair, and after the application step, a heating step of heating the hair. The method for treating hair according to another embodiment of the present invention is a method for treating hair including, subsequent to the application step, a washing step of washing off the hair treatment composition from the hair, and a heating step of heating the hair from which the hair treatment composition has been washed off.

The heating temperature in the heating step is not limited as long as the effects of the hair treatment composition of the present invention can be provided. The heating step is preferably a step of heating the hair in such a manner that the surface temperature of the hair is 40°C to 60°C, 70°C, 80°C, 90°C, 100°C, 110°C, 120°C, 130°C, 140°C, 150°C, 160°C, 170°C, or 180°C, more preferably a step of heating the hair in such a manner that the surface temperature of the hair is 40°C to 60°C.

The method for treating hair of the present invention is advantageous in that the effects can be provided even if the surface temperature of the hair is 40°C to 60°C. This temperature range is a heating temperature range for daily hairdressing at home, and the risk of damaging the hair is low. Thus, in the present invention, the hair treatment effects of the present invention can be obtained without using a specialized apparatus.

The method for treating hair according to another embodiment of the present invention is intended for one or more selected from the group consisting of hair straightening, improvements in hair quality during dyeing and bleaching, improvements in hair quality during a wave perm and a straight perm, shampooing, and hair treatment.

In one embodiment of the hair treatment composition of the present invention, a hair quality improver contains the hair treatment composition and is used for the purpose of improving hair quality during perming. In another embodiment, the hair quality improver is used not as a primary agent or a secondary agent for a wave perm or a straight perm but as an intermediate agent. Specifically, the primary agent is applied to hair, and after a sufficient reducing action is obtained on the hair, the hair treatment composition of the present invention is applied. At the time of application, the hair treatment composition of the present invention may be applied alone, or the hair treatment composition of the present invention and the primary agent may be mixed and applied. The applied hair is allowed to stand. The primary agent and the hair treatment composition of the present invention are washed away. The hair is dried by heating, and if necessary, further heated with, for example, a hair iron. Then the secondary agent is applied. As a result, the finished hair is soft. Even if the hair is repeatedly washed with shampoo after the perming treatment, the state of the strong and soft hair is maintained.

In another embodiment, the present invention provides a hair straightening agent containing the hair treatment composition. In the case of hair straightening, the hair straightening agent is applied to hair singly or as a mixture with a primary agent for hair straightening, allowed to stand, and washed away. The hair dried by heating, and if necessary, further heated with, for example, a hair iron. Then a secondary agent is applied. As a result, the finished hair is soft and free of twisting. Even if the hair is repeatedly washed with shampoo after the hair straightening treatment, the state of the strong and soft hair is maintained.

In another embodiment, the present invention provides a hair quality improver for use in dyeing or bleaching, the hair quality improver containing the hair treatment composition. The hair quality improver is mixed with a primary agent and a secondary agent containing an oxidation dye, or mixed with a liquid mixture of the primary agent and the secondary agent, applied to the hair, allowed to stand, and washed away. The hair is dried by heating, and if necessary, further heated with, for example, a hair iron. As a result, an unpleasant odor immediately after dyeing and bleaching is suppressed, and the finished hair can be highly elastic and soft. Damage to the hair during bleaching is reduced, and twisting of the hair is reduced. That is, the present invention may provide a hair dye or a bleaching agent containing the hair treatment composition.

In another embodiment, the present invention provides a shampoo agent and a hair treatment agent containing the hair treatment composition. Each of the shampoo agent and the hair treatment agent is applied to hair singly or as a mixture with an existing shampoo agent or hair treatment agent, allowed to stand, and washed away. The hair is dried by heating, and if necessary, further heated with, for example, a hair iron. As a result, the finished hair can be highly elastic and soft. Thereafter, even if the hair is washed with shampoo, the elasticity and softness of the hair are maintained.

In another embodiment of the hair treatment composition of the present invention, the present invention provides a hair styling agent (hair dressing) containing the hair treatment composition. The hair styling agent of the present invention can highly maintain the elasticity and softness of hair in a styled state, and can impart a high hair styling effect.

In another embodiment of the hair treatment composition of the present invention, the present invention provides a hair tonic and/or a hair restorer containing the hair treatment composition. The hair tonic and/or hair restorer of the present invention can impart a high hair growing effect and/or hair restoring effect by keeping the elasticity and softness of the hair high.

### EXAMPLES

While the present invention will be described in more detail with reference to examples, the present invention is not limited to the following examples.

### 1. Preparation of Hair Treatment Composition of Present Invention

As Experimental example 1, a hair treatment composition containing a complex of muconic acid and cysteamine was prepared. Among the three isomers of muconic acid, trans-trans muconic acid was used. First, 1.42 g of muconic acid was added to 10 g of purified water, and then 3 g of aqueous ammonia was added thereto for pH adjustment. The mixture was heated to 80°C. After ensuring the dissolution of muconic acid, 0.77 g of cysteamine was added. The mixture was heated to boiling (up to 100°C) while mixing. The boiling state (100°C) was maintained for 3 minutes to prepare a complex composed of muconic acid and cysteamine. Heating was stopped, and then the mixture was allowed to stand to normal temperature. After ensuring that there was no undissolved residue, the pH of the mixture was adjusted to about 7.0 with aqueous ammonia. Purified water was added to adjust the final concentration of muconic acid and cysteamine to 10% by mass.

As Experimental example 2, a hair treatment composition containing a complex of adipic acid and cysteamine was prepared. First, 1.46 g of adipic acid was added to 10 g of purified water, and 3 g of aqueous ammonia was added for pH adjustment. The mixture was heated to 80°C. After ensuring the dissolution of adipic acid, 0.77 g of cysteamine was added. The mixture was heated to boiling (up to 100°C) while mixing. The boiling state (100°C) was maintained for 3 minutes to prepare a complex composed of adipic acid and cysteamine. Heating was stopped, and then the mixture was allowed to stand to normal temperature. After ensuring that there was no undissolved residue, the solution was adjusted to a pH of about 7.0 with aqueous ammonia. Purified water was added to adjust the final concentration of adipic acid and cysteamine to 10% by mass.

As Experimental example 3, a hair treatment composition was prepared using muconic acid and thioglycerol. Preparation was performed under the same conditions as in Experimental example 1, except that 1.08 g of thioglycerol was added instead of cysteamine.

As Experimental example 4, a hair treatment composition was prepared using adipic acid and thioglycerol. Preparation was performed under the same conditions as in Experimental example 2, except that 1.08 g of thioglycerol was added instead of cysteamine.

As Experimental example 5, a hair treatment composition was prepared using muconic acid and glyceryl thioglycolate. Preparation was performed under the same conditions as in Experimental example 1, except that 1.66 g of glyceryl thioglycolate was added instead of cysteamine.

As Experimental example 6, a hair treatment composition was prepared using adipic acid and glyceryl thioglycolate. Preparation was performed under the same conditions as in Experimental example 2, except that 1.66 g of glyceryl thioglycolate was added instead of cysteamine.

As Experimental example 7, a hair treatment composition was prepared using adipic acid and ammonium thioglycolate. Preparation was performed under the same conditions as in Experimental example 2, except that 2.18 g of a 50% by weight of aqueous solution of ammonium thioglycolate was added instead of cysteamine.

As Comparative example 1, a hair treatment composition was prepared using fumaric acid and cysteamine. Preparation was performed under the same conditions as in Experimental example 2, except that 1.16 g of fumaric acid was added instead of adipic acid.

As Comparative example 2, a hair treatment composition was prepared using succinic acid and cysteamine. Preparation was performed under the same conditions as in Experimental example 2, except that 1.18 g of succinic acid was added instead of adipic acid.

### 2. Evaluation of Hair Treatment Composition of Present Invention (1): Evaluation of Wave-forming power When Heat Treatment Is Performed and its Persistence

The hair treatment composition of the present invention was evaluated. A bundle of 30 to 40 human healthy hairs (average length: 25 cm) was made, and the bundle of hairs was fixed to a plastic measurement comb by winding the hairs in a zigzag fashion. According to the Kirby method (reference: Donald H. Kirby, Drug and Cosmetic Industry, 80 (3), 314-315, 397-400 (1957)), the hair was immersed in a primary perming agent (pH: 9.2), which was a 10% by mass aqueous solution of ammonium thioglycolate, for 10 minutes, and then rinsed with running water at 40°C for 30 seconds. Thereafter, the hair was immersed for 2 minutes in the hair treatment composition of Experimental example 1, Experimental example 2, or Comparative example 1, taken out, and wiped with a towel to remove water on the hair.

The hair fixed to the measurement comb was subjected to a heat treatment at 40°C, 60°C, or 80°C. Hot air was applied to the hair with a hair dryer for 3 minutes in such a manner that the surface temperature of the hair fixed to the measurement comb was 40°C, 60°C, or 80°C, and then the hair was allowed to stand at a normal temperature of 15°C to 20°C for 7 minutes. The hair was immersed in a secondary agent composed of a 6% by mass aqueous solution of sodium bromate for 10 minutes. The hair was taken out and removed from the measurement comb and left to dry at 15°C to 20°C.

The wave-forming power on hair under each condition was measured. With respect to the hair waves formed by the treatment at each temperature, the wave-forming power is expressed as the distance between the peak of one wave and the peak of a wave next to the wave. A shorter distance indicates a stronger wave-forming power on the hair.

At the same time, the feel of the hair immediately after the treatment was evaluated and scored. The case where the feel was worse than before the treatment was scored as 0. The case where the feel was not changed from before the treatment was scored as 1. The case where the feel was slightly improved compared with before the treatment was scored as 2. The case where the feel was clearly good compared with before the treatment was scored as 3. The case where the feel was markedly good compared with before the treatment was scored as 4.

In addition, the persistence of wave formation against shampoo washes was evaluated using the following method. Hair that had been waved was hand-washed with a 2% by weight aqueous solution of a shampoo mainly containing sodium laureth sulfate (hereinafter, referred to as an "aqueous shampoo solution"). The hair was rinsed with running water at 40°C for 30 seconds, then wiped with a towel to remove water on the hair, and dried by blowing with hot air at 80°C using a hair dryer from a distance of 30 cm or more. This treatment was repeated to examine whether the feel obtained immediately after wave formation changed. The number of shampoo washes at which the wave-forming power is lost was determined. The wave-forming power was determined to be lost when the peak-to-peak distance of the wave was two or more times the distance immediately after formation.

In the case of high persistence to the shampoo washes, hair was treated under accelerated deterioration conditions. That is, the hair was immersed in the aqueous shampoo solution at 40°C for 2 hours, rinsed with running water at 40°C for 30 seconds, then wiped with a towel to remove water on the hair, and dried by blowing with hot air at 80°C using a hair dryer from a distance of 30 cm or more. This treatment was determined to have the same effect on the hair as the 28 shampoo washes described above. This accelerated deterioration treatment was combined with the above-described ordinary shampoo washing treatment, and the number of shampoo washes at which the wave-forming power was lost was determined.

Table 1 presents the evaluation results. In Experimental example 1, Experimental example 2, and Comparative example 1, a good wave-forming power of 20 mm or less was provided under any conditions. In Comparative example 1, in the case of the heat treatment in which the surface temperature of the hair was set to 60°C, the effect was maintained for 29 shampoo washes, and in the case of the heat treatment in which the surface temperature of the hair was set to 80°C, the effect was maintained for 57 shampoo washes. In Experimental example 1, in the case of the heat treatment in which the surface temperature of the hair was set to 60°C, the persistence was maintained for 38 or more shampoo washes. In Experimental example 2, in the case of the heat treatment in which the surface temperature of the hair was set to 40°C, the persistence was maintained for 38 or more shampoo washes. That is, in Experimental example 1 and Experimental example 2, the persistence obtained in the heat treatment in which the surface temperature of the hair was set to 40°C to 60°C was higher than the persistence obtained in the heat treatment at 40°C to 60°C in Comparative example 1, and was comparable to the persistence obtained in the heat treatment at 80°C in the comparative example.

In the experimental examples and the comparative examples, the feel immediately after wave formation was studied. The feel of the hair was improved under any of the conditions, compared with before the treatment. In Comparative example 1, in the treatment in which the surface temperature of the hair was 60°C or lower, the feel of the hair was not permanent and was semi-permanent in that the feel was lost by shampoo washes, and the feel was not permanent unless the treatment in which the surface temperature was 80°C was performed. In Experimental example 1, in the heat treatment in which the surface temperature of the hair was set to 60°C, the feel was permanently obtained when the hair was washed with the shampoo. In Experimental example 2, in the heat treatment in which the surface temperature of the hair was set to each of 40°C and 60°C, the feel was permanently obtained when the hair was washed with the shampoo. The results indicated that in Experimental example 1 and Experimental example 2, the marked hair treatment effects were provided by the treatment at a temperature lower than that in Comparative example 1.

**[Table 1]**

| | Experimental Example 1 | | | Experimental Example 2 | | | Comparative Example 1 | | |
|---|---|---|---|---|---|---|---|---|---|
| Surface Temperature of the Hair (°C) | 40 | 60 | 80 | 40 | 60 | 80 | 40 | 60 | 80 |
| Wave-Forming Power (mm) | 20 | 18 | 18 | 19 | 17 | 17 | 16 | 16 | 16 |
| Persistence of Wave Formation | 26~ 27 Times | 38 Times | 38 Times or More | 38 Times or More | 57 Times or More | 57 Times or More | 20 Times | 29 Times | 57 Times |
| Feel Obtained Immediately after Wave Formation Changed | Soft | Feel Good and Soft | Extremely Feel Good and Soft | Soft and Strong | Feel Good, Strong and Shiny | Feel Good, Strong and Shiny | Soft | Soft | Soft and Slightly Strong |
| Feel Score | 2 | 3 | 3 | 3 | 3 | 3 | 2 | 2 | 3 |
| Persistence of Feel as above | Semi-Permanent | Permanent | Permanent | Permanent | Permanent | Permanent | Semi-Permanent | Semi-Permanent | Permanent |

### 3. Evaluation of Hair Treatment Composition of Present Invention (2): Evaluation of Wave-forming power When Treatment Is Performed at Normal Temperature and its Persistence

The wave-forming power and its persistence were evaluated when hair was treated at normal temperature. In the same manner as in "2.", hair fixed to the measurement comb was immersed for 2 minutes in each of the hair treatment compositions of Experimental examples 1 to 7 and Comparative examples 1 and 2. In a blank test, the hair was immersed in purified water for 2 minutes. The hair was taken out and wiped with a towel to remove water on the hair. The hair fixed to the measurement comb was allowed to stand at 15°C to 20°C for 10 minutes and immersed in a secondary agent composed of a 6% by mass aqueous solution of sodium bromate for 10 minutes. The hair was taken out, and the measurement comb was removed therefrom. The hair was dried at 15°C to 20°C.

Tables 2 and 3 present the evaluation results. In each of Experimental examples 1 to 6, a good wave-forming power of 20 mm or less was obtained on the hair under any condition, and the wave was maintained even after 5 to 10 shampoo washes. In Experimental examples 3 and 4, the feel of the hair was unchanged from before the treatment. In Experimental examples 1 and 2 and Experimental examples 5 and 6, the feel of the hair was improved, compared with before the treatment. The feel of the hair obtained in each example was semi-permanent.

**[Table 2]**

| | Blank Test | Experimental Example 1 | Experimental Example 2 | Experimental Example 3 | Experimental Example 4 |
|---|---|---|---|---|---|
| Wave-Forming Power (mm) | 18 | 14 | 16 | is | 16 |
| Persistence of Wave Formation | 5 Times | 10 Times | 10 Times | 5 Times | 8~9 Times |
| Feel Obtained Immediately after Wave Formation Changed | - | Extremely Feel Good and Extremely Soft | Firm | Soft | Slightly Hard |
| Feel Score | 1 | 2 | 2 | 1 | 1 |
| Persistence of Feel as above | - | Semi-Permanent | Semi-Permanent | Semi-Permanent | Semi-Permanent |

**[Table 3]**

| | Experimental Example 5 | Experimental Example 6 | Experimental Example 7 | Comparative Example 1 | Comparative Example 2 |
|---|---|---|---|---|---|
| Wave-Forming Power (mm) | 16 | 16 | 21 | 14 | 21 |
| Persistence of Wave Formation | 10 Times | 10 Times | 1 Time | 10 Times | 1 Time |
| Feel Obtained Immediately after Wave Formation Changed | Soft | Slightly Hard | No Effect | Feel Good and Soft | No Effect |
| Feel Score | 2 | 2 | 1 | 2 | 2 |
| Persistence of Feel as above | Semi-Permanent | Semi-Permanent | Single Time | Semi-Permanent | Short Time |

### 4, Evaluation of Hair Treatment Composition of Present Invention (3): Evaluation of Curl-forming power and its Persistence

Thirty to forty human healthy hairs (average length: 25 cm) were immersed in a primary perming agent (pH: 9.2), which was a 10% by mass aqueous solution of ammonium thioglycolate, for 10 minutes, and rinsed with running water at 40°C for 30 seconds. Thereafter, the hair was immersed in each of the hair treatment compositions of Experimental example 1, Experimental example 2, Experimental example 3, Experimental example 4, Experimental example 5, Experimental example 6, and Experimental example 7 for 2 minutes. The hair of a blank test was immersed in purified water for 2 minutes. The hair was rinsed with running water at 40°C for 30 seconds and wiped with a towel to remove excess water. The hair wound three times around a cylindrical hair iron having a diameter of 25 mm was heated with the hair iron for 2 minutes after the surface temperature of the hair reached 180°C to form a curl, and then removed from the hair iron and allowed to stand at room temperature for 7 minutes. Thereafter, the hair was immersed in a secondary perming agent composed of a 6% by mass aqueous solution of sodium bromate for 10 minutes, taken out, and then dried at normal temperature.

The diameter or circumference of the curl of the hair was measured and this was evaluated as the curl-forming power. A smaller measured value indicates a stronger curl-forming power on the hair. The persistence of curl formation against shampoo washes was studied, and the feel of the hair immediately after the hair treatment was evaluated and scored in the same manner as described above.

Tables 4 and 5 present the evaluation results. In Experimental example 3, the curl-forming power was not provided on the hair. In Experimental examples 1 and 2 and Experimental examples 4 to 7, the curl-forming power was provided. In particular, the curl-forming power in each of Experimental examples 1 and 2 was maintained even after 85 or more shampoo washes and thus had high persistence. The feel immediately after the formation of the curl was improved under any of the conditions, but the feel was particularly markedly improved in Experimental examples 1 and 2.

The feel of the hair obtained in each of Experimental example 1 and Experimental example 2 was permanent. The persistence of the effect was noticeable even compared with other experimental examples and comparative examples. The feel in each of Experimental example 1 and Experimental example 2 did not deteriorate at all even after 100 or more shampoo washes.

**[Table 4]**

| | Blank Test | Experimental Example 1 | Experimental Example 2 | Experimental Example 3 | Experimental Example 4 |
|---|---|---|---|---|---|
| Curl-Forming Power [Circumference] (mm) | 134 | 120 | 87 | N.A. | 128 |
| Persistence of Curl Formation | 3 Times | 85 Times or More | 85 Times or More | N.A. | 10 Times |
| Feel Obtained Immediately after Curl Formation Changed | - | Extremely Feel Good and Extremely Soft | Shiny, Feel Good and Firm | Soft | Strength of Hair Surface Slightly Increased |
| Feel Score | 1 | 4 | a | 2 | 2 |
| Persistence of Feel as above | Temporary | Permanent | Permanent | Temporary | Semi-Permanent |

**[Table 5]**

| | Experimental Example 5 | Experimental Example 6 | Experimental Example 7 | Comparative Example 1 | Comparative Exampl e 2 |
|---|---|---|---|---|---|
| Curl-Forming Power [Circumference] (mm) | 130 or More | 120 | 160 | 108 | 128 |
| Persistence of Curl Formation | 5 Times | 29 Times | 2 Times | 57 Times | 1 Time |
| Feel Obtained Immediately after Curl Formation Changed | Soft | Strength of Hair Surface Slightly Increased | Gloss was Observed | Feel Good, Soft and Curl-Forming Power was Strong | Slightly Hard |
| Feel Score | 2 | 3 | 2 | 4 | 2 |
| Persistence of Feel as above | Semi-Permanent | Semi-Permanent | Temporary | Permanent | Temporary |

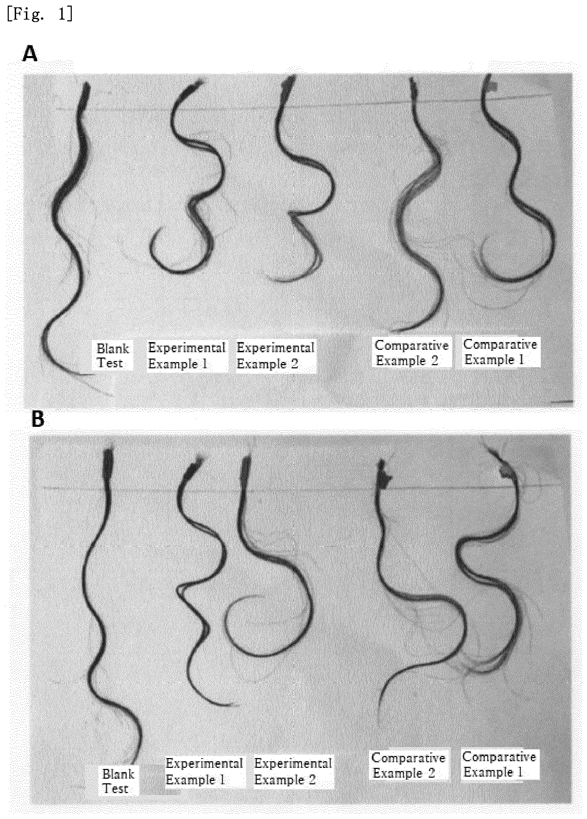

Fig. 1A is a photograph of the hair immediately after treatment with each of the compositions of Experimental example 1, Experimental example 2, Comparative example 1, and Comparative example 2 and, as a blank test, further with purified water in place of the experimental examples and comparative examples, under the same conditions as described above. Fig. 1B is a photograph of the hair after shampoo treatment corresponding to 87 times, in other words, 3 times of accelerated deterioration treatment and 3 times of ordinary shampooing. For heating the hair, a hot rod having a diameter of 15 mm was used instead of the hair iron having a diameter of 25 mm.

As presented in Fig. 1A, in Experimental example 1 (indicated as "Ex. 1" in the figure), Experimental example 2 (indicated as "Ex. 2" in the figure), Comparative example 1 (indicated as "Comp. 1" in the figures), and Comparative example 2 (indicated as "Comp. 2" in the figure), the curl-forming power on the hair immediately after the treatment was markedly strong as compared with the blank test (indicated as "Blank" in the figure). In Comparative example 1 and Comparative example 2, the manageability of the hair ends was lost by the shampoo corresponding to 87 times, whereas the hair treated with each of Experimental example 1 and Experimental example 2 maintained the same quality as before the shampoo even after the shampoo corresponding to 87 times.

### 5, Evaluation of Hair Treatment Composition of Present Invention (4): Evaluation of Hair-Straightening Power and its Persistence

Thirty to forty curly hairs (average length: 25 cm) of Japanese women were immersed in a primary perming agent (pH: 9.2) of a 10% by mass aqueous solution of ammonium thioglycolate for 10 minutes, and rinsed with running water at 40°C for 30 seconds. Thereafter, the hair was immersed for 2 minutes in the hair treatment composition of Experimental example 1 or Experimental example 2, rinsed with running water at 40°C for 30 seconds, and wiped with a towel to remove excess water. The hair was straightened with a flat iron from the root to the hair tip over 1 minute while pulling the hair little by little so as to remove the twist from the root of the hair. Furthermore, the hair was passed once through an iron at a uniform speed over 3 seconds from the root to the hair tip. Heat was applied again from the root of the hair. The hair was left at normal temperature for 7 minutes. Thereafter, the hair was immersed in a secondary perming agent composed of a 6% by mass aqueous solution of sodium bromate for 10 minutes, taken out, and then dried at normal temperature.

The results indicated that the finished hair treated with each of Experimental examples 1 and 2 was soft. In particular, in Experimental example 2, the hair was both soft and strong, and the surface of the hair was shiny. Measurement of the persistence of the hair-straightening power against shampoo washes revealed a high persistence of 85 times or more in both Experimental examples 1 and 2.

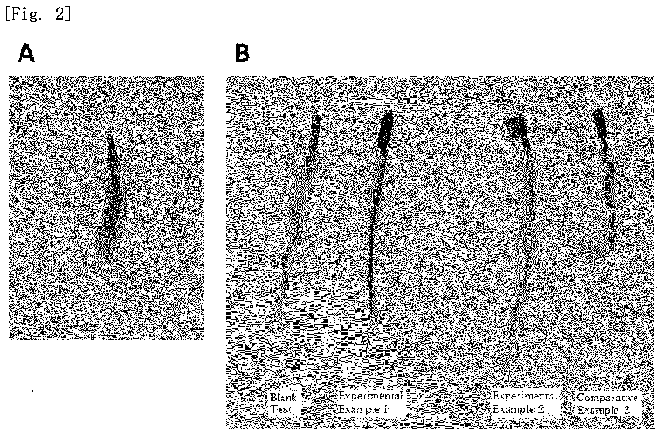

Fig. 2 presents the results of another hair straightening test. The hair of Ethiopian women having a strong frizz tendency was immersed in a primary perming agent (pH: 9.2) of a 10% by mass aqueous solution of ammonium thioglycolate for 30 minutes, and rinsed with running water at 40°C for 30 seconds. Thereafter, the hair was immersed in each of the hair treatment compositions of Experimental example 1, Experimental example 2, and Comparative example 2 for 3 minutes. As a blank test, the hair was treated under the same conditions using purified water in place of the experimental examples and the comparative examples. The hair was rinsed with running water at 40°C for 30 seconds and wiped with a towel to remove excess water. The water was further removed with a low-temperature dryer, and the hair was slowly stretched with an iron at 180°C.

Fig. 2A is a photograph illustrating the state of curly hair (20 to 30 hairs, average length: 15 cm) of Ethiopian women before treatment. Fig. 2B is a photograph illustrating the state of hair after the treatment. In Comparative example 2 (indicated as "Comp. 2" in the figure), the state was the same as the hair used in the blank test (indicated as "Blank" in the figure). In Experimental example 1 (indicated as "Ex. 1" in the figure) and Experimental example 2 (indicated as "Ex. 2" in the figure), the frizzy hair was straightened. In particular, in Experimental example 1, the hair tips were well gathered, and an ideal hair straightening effect was provided.

### 6, Evaluation of Hair Treatment Composition of Present Invention (5): Evaluation as Hair Treatment Agent at each Treatment Temperature for Hair

The hair treatment effects of the compositions of Experimental examples 1 and 2 on human healthy hair or bleached hair were examined. A bundle of 30 to 40 human healthy hairs or bleached hairs (average length: 25 cm) was immersed for 3 minutes in a liquid (pH: 7) prepared by diluting the composition of Experimental example 1, Experimental example 2, or Comparative example 2 in such a manner that the concentration of the dicarboxylic acid and the thiol was 0.5% by mass. Excess water was wiped off. The hair was subjected to treatment such that the surface temperature of the hair was 20°C, 60°C, 80°C, or 120°C.

For the treatment in which the hair was brought to 20°C, the hair was wrapped around a 15-mm hot rod with no gaps and allowed to stand at 20°C (room temperature) for 10 minutes. Any excess water was wiped off with a towel. The hair was allowed to stand for another minute before the hair was removed from the hot rod. For the treatment in which the hair was brought to 60°C or 80°C, the hair was wound around a 15-mm hot rod with no gaps and heated for 10 minutes in such a manner that the hair surface was brought to 60°C or 80°C. The hair was allowed to stand for another minute to cool the hot rod and removed from the hot rod. For the treatment in which the hair was brought to 120°C, the hair was wound three times around a cylindrical hair iron having a diameter of 25 mm and heated in such a manner that the hair was held at about 180°C for 10 minutes, thereby forming a curl. Then the hair was removed from the hair iron and allowed to stand at normal temperature for 1 minute.

Each hair was immersed in an aqueous shampoo solution at 40°C for 10 seconds, then rinsed with running water at 40°C for 10 seconds, and wiped with a towel to remove water. The effect on the hair was evaluated on the basis of overall findings including the curl formation rate, the persistence of curl formation, and the feel. The curl formation rate was calculated as follows: The distance (curl diameter) from the bending start position, serving as a starting point, on the root side of the hair bundle in a curled state to the tip of the hair having the second longest hair in the hair bundle was measured. Furthermore, the distance (hair length) from the starting point to the tip of the relevant hair in a stretched state of the hair bundle was measured. The curl formation rate was calculated from (hair length - curl diameter)/hair length × 100.

To evaluate the persistence of curl formation, the hair bundle was immersed in the aqueous shampoo solution, rinsed with water, wiped with a towel to remove excess water, and was left to dry at room temperature. This operation was defined as one treatment cycle. The number of treatment cycles at which the formed curl was no longer maintained (less than one turn of the hair bundle) was measured. Under the condition that the bleached hair was set at 80°C, the hair was evaluated according to the feel score described above.

Table 6 presents the evaluation results when the healthy hair was used. Table 7 presents the evaluation results when the bleached hair was used. In Experimental example 1 and Experimental example 2, in the treatment in which the surface temperature of the hair was set to 40°C to 180°C, a higher curl formation rate was measured than that in the comparative example. In addition, in Experimental example 1 and Experimental example 2, in the treatment in which the surface temperature of the hair was set to 20°C to 80°C, the persistence of the curl formation against at least two or more shampoo actions was provided. In Comparative example 2, the curl formation was lost by one shampoo wash under any temperature condition. In Experimental example 1 and Experimental example 2, the feel of the bleached hair was improved. As described above, the effect of the hair treatment agent containing the composition of the present invention was clarified.

**[Table 6]**

| | Experimental Example 1 | | | | Experimental Example 2 | | | | Comparative Example 2 | | | |
|---|---|---|---|---|---|---|---|---|---|---|---|---|
| Hair Treatment Temperature (°C) | 20 | 60 | 80 | 180 | 20 | 60 | 80 | 180 | 20 | 60 | 80 | 180 |
| Curl Formation Rate (%) | 8.2 | 13.5 | 14.0 | 20.8 | 6.9 | 11.2 | 10.9 | 15.6 | 4.3 | 9.0 | 9.9 | 8.2 |
| Persistence (Number of Times) | 2 | 2 | 2 | 1 | 3 | 3 | 3 | 2 | 1 | 1 | 1 | 1 |
| Finding | Soft | Soft | Soft and Glossy of Hair Surface | Soft, Glossy of Hair Surface and Extremely Feel Good | Strong | Strong and Glossy of Hair Surface | Strong and Glossy of Hair Surface | Strong and Glossy of Hair Surface | Strong and Hard | Strong and Hard | Strong and Hard | Strong and Hard |

**[Table 7]**

| | Experimental Example 1 | | | | Experimental Example 2 | | | | Comparative Example 2 | | | |
|---|---|---|---|---|---|---|---|---|---|---|---|---|
| Hair Treatment Temperature (°C) | 20 | 60 | 80 | 180 | 20 | 60 | 80 | 180 | 20 | 60 | 80 | 180 |
| Curl Formation Rate (%) | 8.9 | 12.5 | 16.5 | 21.0 | 6.3 | 14.0 | 14.0 | 20.3 | 3.4 | 8.9 | 9.3 | 6.7 |
| Persistence Number of Times) | 2 | 2 | 2 | 1 | 3 | 3 | 4 | 2 | 1 | 1 | 1 | 1 |
| Feel Score | - | - | 3 | - | - | - | 3 | - | - | - | 2 | - |
| Finding | Soft | Soft and Glossy of Hair Surface | Soft and Glossy of Hair Surface | Soft, Glossy of Hair Surface and Extremely Feel Good | Strong | Strong and Glossy of Hair Surface | Strong and Glossy of Hair Surface | Strong and Glossy of Hair Surface | Strong and Hard | Strong and Hard | Strong and Hard | Strong and Hard |

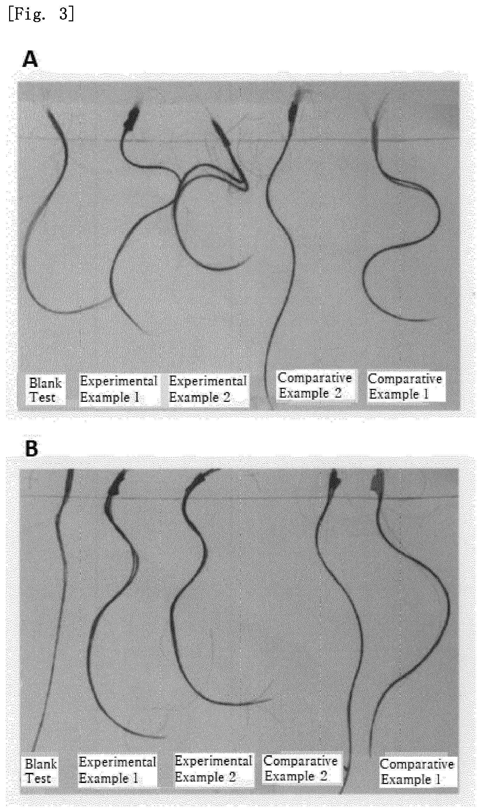

Among the conditions described in Table 7, Fig. 3A presents a photograph of the bleached hair immediately after the treatment at 60°C. Fig. 3B presents a photograph of the bleached hair shampooed after treatment. In addition to Experimental example 1 (indicated as "Ex. 1" in the figure), Experimental example 2 (indicated as "Ex. 2" in the figure), and Comparative example 2 (indicated as "Comp. 2" in the figure), Comparative example 1 (indicated as "Comp. 1" in the figure) was used for the treatment. For a blank test, purified water in place of the experimental examples and comparative examples was used (indicated as "Blank" in the figure). The bleached hair in the blank test was damaged from bleaching. The bleached hair treated with each of Experimental example 1 and Experimental example 2 was soft and strong, and the curl was maintained even after shampooing. In Comparative example 1 and Comparative example 2, the curl of the bleached hair disappeared by shampooing.

### 7, Evaluation of hair treatment composition of present invention (6): Evaluation of Composition, as Hair Treatment Agent, Prepared in Mixing Step Under each Temperature Condition

The effect, as hair treatment agents, of the compositions prepared in the mixing step of mixing the dicarboxylic acid and the thiol at 20°C (normal temperature), 60°C, or 80°C (Experimental examples 8 to 14) and the compositions prepared in the step of mixing them at 100°C (Experimental examples 1 and 2) were examined.

In each of Experimental examples 8 to 11, the hair treatment composition was prepared using muconic acid and cysteamine. First, 1.42 g of muconic acid was added to 10 g of purified water, followed by the addition of 0.77 g of cysteamine. In Experimental examples 8 and 9, mixing was performed at 20°C (normal temperature). In Experimental example 10, the mixture was heated to 60°C, and mixing was performed. In Experimental example 11, the mixture was heated to 80°C, and mixing was performed. When muconic acid and cysteamine were completely dissolved in water, heating was stopped in Experimental examples 10 and 11, and the mixture was left to normal temperature. Purified water and, if necessary, aqueous ammonia were added to adjust the pH to about 7. Then purified water was added to adjust the final concentration of muconic acid and cysteamine to 10% by weight. It was difficult to adjust the pH to 7 under the condition of stirring at 20°C; thus, the pH was adjusted to 6.5 in Experimental example 8, and the pH was adjusted to 8 in Experimental example 9.

In Experimental examples 12 to 14, hair treatment compositions were prepared using adipic acid and cysteamine. First, 1.46 g of adipic acid was added to 10 g of purified water, followed by the addition of 0.77 g of cysteamine. In Experimental example 12, mixing was performed at 20°C (normal temperature). In Experimental example 13, the mixture was heated to 60°C, and mixing was performed. In Experimental example 14, the mixture was heated to 80°C, and mixing was performed. When adipic acid and cysteamine were completely dissolved in water, heating was stopped in Experimental examples 13 and 14, and the mixture was left to normal temperature. The solution was adjusted to a pH of about 7.0 with aqueous ammonia. Purified water was added to adjust the final concentration of adipic acid and cysteamine to 10% by mass.

With respect to, in addition to Experimental examples 8 to 14, Experimental example 1 in which the complex obtained by mixing muconic acid and cysteamine at 100°C was contained and Experimental example 2 in which the complex obtained by mixing adipic acid and cysteamine at 100°C was contained, the hair treatment effects of those compositions on human healthy hair or bleached hair were examined. A liquid prepared by diluting the composition of each experimental example in such a manner that the concentration of the dicarboxylic acid and the thiol was 0.5% by mass was used. The healthy hair and the bleached hair were evaluated in the same way as in "5.", except that the hair was heated so as to have a surface temperature of 80°C. Table 8 presents the evaluation results when the healthy hair was used. Table 9 presents the evaluation results when the bleached hair was used. Regarding the compositions using muconic acid (Experimental examples 8 to 11 and 1), the feel of the hair was superior in the experimental examples in which the compositions were prepared by mixing at 60°C or higher. Regarding the compositions using adipic acid (Experimental examples 12 to 14 and 2), the persistence of the curl and the feel of the hair were superior in Experimental examples in which the compositions were prepared by mixing at 60°C or higher. As described above, in the experimental examples in which the mixing step was performed at a higher temperature, the persistence of the curl and the feel score tended to be superior. This suggested that a higher temperature in the mixing step would promote the formation of the complex.

**[Table 8]**

| | Experimental Example | 8 | 9 | 10 | 11 | 1 | 12 | 13 | 14 | 2 |
|---|---|---|---|---|---|---|---|---|---|---|
| Curl Formation Rate (%) | | 10.1 | 10.1 | 12.6 | 15.3 | 14.0 | 9.9 | 11.2 | 10.9 | 15.6 |
| Persistence (Number of Times) | | 1 | 1 | 1 | 1 | 2 | 1 | 2 | 2 | 3 |
| Feel Score | | 2 | 2 | 3 | 3 | 4 | 2 | 3 | 3 | 4 |
| Finding | | Not Soft | Not Soft | Soft | Soft | Soft, Glossy of Hair Surface and Extremely Feel Good | Elastic was obtained | Strong | Strong and Glossy of Hair Surface | Strong and Extremely Glossy of Hair Surface |

**[Table 9]**

| | Experimental Example | 8 | 9 | 10 | 11 | 1 | 12 | 13 | 14 | 2 |
|---|---|---|---|---|---|---|---|---|---|---|
| Curl Formation Rate (%) | | 14.2 | 13.9 | 16.2 | 17.5 | 16.5 | 10.5 | 14.0 | 14.0 | 20.3 |
| Persistence of Curl Formation (Number of Times) | | 1 | 1 | 1 | 1 | 2 | 1 | 2 | 2 | 3 |
| Feel Score | | 2 | 2 | 3 | 3 | 4 | 2 | 3 | 3 | 4 |
| Finding | | Not Soft | Not Soft | Soft | Soft | Soft, Glossy of Hair Surface and Extremely Feel Good | Elastic was obtained | Strong | Strong and Glossy of Hair Surface | Strong and Extremely Glossy of Hair Surface |

### 8, Evaluation of Hair Treatment Composition of Present Invention (7)

With respect to Experimental examples 2 and 12, the wave-forming power and its persistence, and the curl-forming power and its persistence were evaluated. The evaluation methods were the same as the above-described methods. Comparisons were made in terms of wave-forming power and its persistence when the hair was heated in such a manner that the surface temperature of the hair was 40°C. Comparison were made in terms of curl-forming power and its persistence when the hair was heated in such a manner that the surface temperature of the hair was 180°C.

Table 10 presents the results. In Experimental example 2, the feel was good when wave formation and curl formation were performed, and the persistence thereof was provided even after multiple shampoo washes. In Experimental example 12, the feel was inferior when wave formation and curl formation were performed, and the persistence thereof after shampoo washes was low, compared with Experimental example 2.

**[Table 10]**

| | Experimental Example 2 | Experimental Example 12 | Experimental Example 2 | Experimental Example 12 |
|---|---|---|---|---|
| Surface Temperature of the Hair (°C) | 40 | 40 | 180 | 180 |
| Wave-Forming Power (mm) | 14 | 15 | | |
| Curl-Forming Power (mm) | | | 90 | 150 or More |
| Persistence of Wave/Curl Formation | Not Exceeding 16 mm in 30 Times | Exceeding 18mm in 30 Times | No Deterioration in 3 Times | Extremely Deterioration in 3 Times |
| Feel Obtained Immediately after Wave/Curl Formation Changed | Feel Good | Damage was Observed in Cuticle | Elastic and Soft | Formed Curl was No Longer Maintained |
| Persistence of Feel as above | Permanent | Semi-Permanent | Permanent | Temporary |

### 9, Evaluation of Hair Treatment Composition of Present Invention (8): Evaluation as Primary Perming Agent in Digital Perm (Perm Using Hot Rod)

As Experimental example 15, a composition for hair was prepared by mixing adipic acid and cysteamine at 40°C. First, 1.46 g of adipic acid was added to 10 g of purified water, followed by the addition of 0.77 g of cysteamine. They were mixed at 40°C. When they were completely dissolved, the resulting solution was left to normal temperature. The solution was adjusted to a pH of about 6.5 with aqueous ammonia. Purified water was added to adjust the final concentration of adipic acid and cysteamine to 10% by mass. Evaluation was made of the effects of the primary perming agent containing each of the compositions of Experimental example 12 in which mixing was performed at 20°C (normal temperature), Experimental example 13 in which mixing was performed at 60°C, Experimental example 14 in which mixing was performed at 80°C, and Experimental example 2 in which mixing was performed at 100°C, in addition to the composition of Experimental example 15.

The composition of each Experimental example was diluted with an aqueous solution containing 10% by mass of ammonium thioglycolate in such a manner that the concentration of adipic acid and cysteamine was 0.5% by mass, and the pH was adjusted to 9. Thereby, primary perming agents were prepared. Thirty to forty human healthy hairs (average length: 25 cm) were immersed in each of the primary agents for 10 minutes, rinsed with running water at 40°C for 20 seconds, wound four times around a hot rod heated to 60°C, and allowed to stand for 10 minutes with the hairs wound with felt to prevent drying. Thereafter, the hair was immersed in a secondary perming agent composed of a 6% by mass aqueous solution of sodium bromate for 10 minutes. The hair was taken out, removed from the hot rod, and dried at normal temperature.

Table 11 presents the evaluation results. In Experimental examples 12 and 15, small damage was observed in the cuticle on the hair surface. In Experimental examples 13, 14, and 2, there was no damage, the feel was good, and shine was also seen on the hair treated with each of Experimental example 15 and Experimental example 2. Regarding the hair treated in each of Experimental examples 13, 14, and 2, the curl-forming power was less than 30 mm in diameter, and the persistence against 33 or more shampoo washes was provided. As described above, the hair treatment compositions of Experimental examples 13, 14, and 2 in which the complexes prepared by mixing adipic acid and cysteamine at 60°C to 100°C were contained were determined to have a marked hair quality improvement effect on hair when used as the primary perming agent, compared with Experimental examples 12 and 15 in which mixing was performed at 20°C to 40°C.

**[Table 11]**

| | Experimental Example12 | Experimental Example 15 | Experimental Example 13 | Experimental Example 14 | Experimental Example 2 |
|---|---|---|---|---|---|
| Feel Obtained Immediately after Curl Formation Changed | Slightly Damage was Observed in the Cuticle | Slightly Damage was Observed in the Cuticle | Feel Good and Slightly Glossy | Feel Good and Slightly Glossy of Hair Surface | Feel Good and Glossy of Hair Surface |
| Curl-Forming Power [Circumference] (mm) | 42 | 35 | 27 | 26 | 24 |
| Persistence of Curl Formation | 2 Times | 4 Times | 33 Times | 50 Times | 57 Times |
| Feel Score | 1 | 1 | 3 | 3 | 3 |
| Persistence of Feel as above | Temporary | Semi-Permanent | Semi-Permanent | Permanent | Permanent |

### 10. Evaluation of Hair Treatment Composition of Present Invention (9): Evaluation of Shampoo Agent Containing Hair Treatment Composition of Present Invention

The effects of shampoo agents containing the hair treatment compositions of the experimental examples were examined. Shampoo agent stock solutions were prepared, each shampoo agent stock solution containing 6% by weight of TEA-lauroyl methylaminopropionate, 3% by weight of lauryl betaine, 1% by weight of cocamide methyl MEA, 1% by weight of cocamide DEA, 0.3% by weight of polyquaternium-10, and 4% by weight of Experimental example 1 or 2. For the blank test, a shampoo agent stock solution containing purified water in place of the experimental example was used.

Each shampoo agent stock solution was added to hot water at about 40°C in such a manner that the concentration was 10% by weight. The mixture was stirred until foaming occurred, thereby providing a shampoo agent. A bundle of 30 to 40 human bleached hairs (average length: 25 cm) was immersed in the shampoo liquid and taken out after 10 seconds. The hair bundle was rubbed by hand for about 10 seconds and rinsed with running water at about 40°C for about 10 seconds. The hair was wiped with a towel to remove water and dried with cold air using a hair dryer.

The hair was wound three times around a cylindrical hair iron having a diameter of 25 mm and heated for 2 minutes after the surface temperature of the hair reached 180°C to form a curl. The curled hair was rinsed with running water at about 40°C for about 10 seconds and wiped with a towel to remove water. When the hair was dried, the curl formation rate was measured by the same method as described above. This was defined as the curl formation rate of the first shampooing. Furthermore, each hair was additionally subjected to the same shampoo treatment twice using the same shampoo agent, and the curl formation rates after the second and third shampooing were measured.

Table 12 presents the results. In the hair for which the shampoo agent containing Experimental example 1 or Experimental example 2 was used, noticeable curls were formed. In the shampoo agent containing Experimental example 1 or Experimental example 2, the curls of the hair became stronger as the number of times of shampooing increased. In the case of using the shampoo agent in which purified water was used in place of the experimental example, almost no curls were formed.

The hair for which the shampoo agent containing Experimental Example 1 or Experimental Example 2 was used was shiny and soft. The feel of these hairs after heating with a hair iron was particularly good. Damage on the hair due to bleaching was not felt on these hairs.

**[Table 12]**

| Curl Formation Rate(%) | Blank Test | Shampoo Agent Containing Experimental Example 1 | Shampoo Agent Containing Experimental Example 2 |
|---|---|---|---|
| First Time | 1.2 | 12.0 | 9.1 |
| Second Time | 1.6 | 20.8 | 13.6 |
| Third Time | 2.1 | 24.0 | 20.2 |

Furthermore, the results of another examination for the effects of the shampoo agent containing the experimental example of the present invention are described. In addition to the shampoo agent stock solution containing Experimental example 1 or Experimental example 2, a shampoo agent stock solution containing Comparative example 1 was prepared in the same manner as described above. As a blank test, a shampoo agent stock solution containing purified water in place of the experimental examples and the comparative example was used. Each shampoo agent stock solution was added to hot water at about 40°C in such a manner that the concentration was 10% by weight. The mixture was stirred until foaming occurred, thereby providing a shampoo agent for hair. A bundle of 30 to 40 human bleached hairs (average length: 25 cm) was immersed in the shampoo liquid, and taken out after 10 seconds. The hair bundle was rinsed with running water at about 40°C for about 10 seconds.

The hair was wound around a hair curler having a diameter of 25 mm, and heated with a hair dryer for 3 minutes in such a manner that the hair surface was brought to about 60°C. The hair was carefully removed from the hair curler, gently showered with hot water at about 40°C, wiped with a towel to remove water, and allowed to stand.

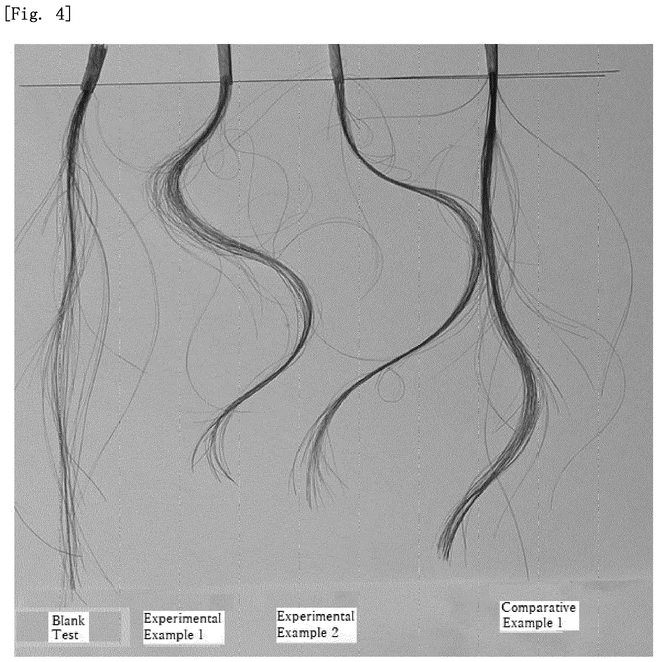

Table 13 presents the curl formation rate of the hair. Fig. 4 is a photograph illustrating the state of the hair. The hair for which the shampoo agent containing Experimental example 1 (indicated as "Ex. 1" in the figure) was used and the hair for which the shampoo agent containing Experimental example 2 (indicated as "Ex. 2" in the figure) was used had an extremely high curl formation rate, compared with the hair of the blank test (indicated as "Blank" in the figure) and the hair for which the shampoo agent containing Comparative example 1 (indicated as "Comp. 1" in the figure) was used, and the curl was maintained even after hot water showering.

**[Table 13]**

| | Blank Test | Shampoo Agent Containing Experimental Example 1 | Shampoo Agent Containing Experimental Example 2 | Shampoo Agent Containing Comparative Example 1 |
|---|---|---|---|---|
| Curl Formation Rate(%) | 0.5 % | 14.1 % | 11.4% | 5.9 % |

### 11. Evaluation of Hair Treatment Composition of Present Invention (10): Verification of Combination of Dicarboxylic Acid and Thiol

Experimental example 11 in which the complex of muconic acid and cysteamine was contained and Experimental example 14 in which the complex of adipic acid and cysteamine was contained were used. In addition, Experimental example 16 in which a complex of (E)-oct-4-ene-1,8-dioic acid, which is a type of octenedioic acid, and cysteamine was contained was prepared. The preparation was performed under the same conditions as in Experimental example 11, except that 1.73 g of (E)-oct-4-ene-1,8-dioic acid was added instead of muconic acid.

Furthermore, Experimental example 17 was prepared using muconic acid and thiolactic acid. Experimental example 18 was prepared using muconic acid and thiomalic acid. Experimental example 17 was prepared under the same conditions as in Experimental example 11, except that 1.06 g of thiolactic acid was added instead of cysteamine. Experimental example 18 was prepared under the same conditions as in Experimental example 11, except that 1.50 g of thiomalic acid was added instead of cysteamine.

The compositions of Experimental examples 11, 14, and 16 were used to verify the hair treatment effects on human healthy hair or bleached hair. A bundle of 30 to 40 human healthy hairs or bleached hairs (average length: 25 cm) was immersed for 3 minutes in a liquid (pH: 7) prepared by diluting the composition of each of the experimental examples in such a manner that the concentration of dicarboxylic acid and thiol was 0.5% by mass, and excess water was wiped off. The hair was wound around a 15-mm hot rod with no gaps and heated for 10 minutes in such a manner that the hair surface was brought to 80°C. The hair was allowed to stand for another minute to cool the hot rod and removed from the hot rod.

Each hair was immersed in an aqueous shampoo solution at 40°C for 10 seconds, then rinsed with running water at 40°C for 10 seconds, and wiped with a towel to remove water. The effect on the hair was evaluated on the basis of overall findings including the curl formation rate, the persistence of curl formation, and the feel. The curl formation rate was calculated as follows: The distance (curl diameter) from the bending start position, serving as a starting point, on the root side of the hair bundle in a curled state to the tip of the hair having the second longest hair in the hair bundle was measured. Furthermore, the distance (hair length) from the starting point to the tip of the relevant hair in a stretched state of the hair bundle was measured. The curl formation rate was calculated from (hair length - curl diameter)/hair length × 100.

To evaluate the persistence of curl formation, the hair bundle was immersed in the aqueous shampoo solution, rinsed with water, wiped with a towel to remove excess water, and was left to dry at room temperature. This operation was defined as one treatment cycle. The number of treatment cycles at which the formed curl was no longer maintained (less than one turn of the hair bundle) was measured.

Table 14 presents the evaluation results when the healthy hair was used. Table 15 presents the evaluation results when the bleached hair was used. In each of Experimental examples 11, 14, and 16, the state of the hair was good, and highly persistent curls of the hair were formed.

**[Table 14]**

| | Experimental Example 11 | Experimental Example 14 | Experimental Example 16 |
|---|---|---|---|
| Curl Formation Rate (%) | 14.0 | 10.9 | 33.1 |
| Persistence (Number of Times) | 5 | 2 | 4 |
| Finding | Strong, Soft and Shiny of Hair Surface | Strong and Shiny of Hair Surface | Strong, Soft and Shiny of Hair Surface |

**[Table 15]**

| | Experimental Example 11 | Experimental Example 14 | Experimental Example 16 |
|---|---|---|---|
| Curl Formation Rate (%) | 42.1 | 14.0 | 43.6 |
| Persistence (Number of Times) | 4 | 2 | 3 |
| Finding | Strong, Soft and Shiny of Hair Surface | Strong and Shiny of Hair Surface | Strong, Soft and Shiny of Hair Surface |

Evaluation as a hair treatment agent was performed in the same manner as above, using Experimental examples 11, 17, and 18 and a blank test (purified water). Table 16 presents the results. In Experimental example 11, marked effects were provided, compared with Experimental examples 17 and 18.

**[Table 16]**

| | Blank Test | Experimental Example 11 | Experimental Example 17 | Experimental Example 18 |
|---|---|---|---|---|
| Finding | No Effect | Strong, Soft and Shiny of Hair Surface | No Effect | No Effect |

Furthermore, using Experimental example 11 in which muconic acid was used, Experimental example 14 in which adipic acid was used, Experimental example 16 in which (E)-oct-4-ene-1,8-dioic acid was used, and Comparative example 1 in which fumaric acid was used, comparisons were made by the same method as in "10." in terms of the effects on human bleached hair. A shampoo stock solution containing each of the compositions of Experimental example 11, Experimental example 14, Experimental example 16, and Comparative example 1 was prepared. As a blank test, a shampoo agent stock solution containing purified water in place of the experimental examples and the comparative example was used. Each shampoo agent stock solution was added to hot water at about 40°C in such a manner that the concentration was 10% by weight. The mixture was stirred until foaming occurred, thereby providing a shampoo agent for hair. A bundle of 30 to 40 human bleached hairs (average length: 25 cm) was immersed in the shampoo liquid, and taken out after 10 seconds. The hair bundle was rinsed with running water at about 40°C for about 10 seconds.

The hair was wound around a hair curler having a diameter of 25 mm, and heated with a hair dryer for 3 minutes in such a manner that the hair surface was brought to about 60°C. The hair was carefully removed from the hair curler, gently showered with hot water at about 40°C, wiped with a towel to remove water, and allowed to stand. The curl formation rate was then measured. Table 17 presents the results. When the composition of each of Experimental examples 11, 14, and 16 was used, a markedly high curl formation rate was obtained, compared with Comparative example 1.

**[Table 17]**

| | Blank Test | Comparative Example 1 | Experimental Example 11 | Experimental Example 14 | Experimental Example 16 |
|---|---|---|---|---|---|
| Curl Formation Rate (%) | 0.5 | 5.9 | 12.3 | 11.4 | 12.2 |

### 12. Evaluation of Hair Treatment Composition of Present Invention (11): Effect of pH in Mixing Step on Hair Treatment Effect of Composition

As Experimental example 19, a hair treatment composition was prepared using muconic acid and cysteamine. Among the three isomers of muconic acid, trans-trans muconic acid was used. First, 1.42 g of muconic acid was added to 10 g of purified water, followed by the addition of aqueous ammonia to adjust the pH to 4.2. The mixture was heated to 80°C. After ensuring the dissolution of muconic acid, 0.77 g of cysteamine was added. The mixture was heated to boiling (up to 100°C) while mixing. The boiling state (100°C) was maintained for 3 minutes to prepare a complex. Heating was stopped, and then the mixture was allowed to stand to normal temperature. After ensuring that there was no undissolved residue, the pH of the mixture was adjusted to about 7.0 with aqueous ammonia. Purified water was added to adjust the final concentration of muconic acid and cysteamine to 10% by mass.

As Experimental example 20, a hair treatment composition was prepared using muconic acid and cysteamine. Among the three isomers of muconic acid, trans-trans muconic acid was used. First, 1.42 g of muconic acid was added to 10 g of purified water, followed by the addition of aqueous ammonia to adjust the pH to 9.0. The mixture was heated to 80°C. After ensuring the dissolution of muconic acid, 0.77 g of cysteamine was added. The mixture was heated to boiling (up to 100°C) while mixing. The boiling state (100°C) was maintained for 3 minutes. Heating was stopped, and then the mixture was allowed to stand to normal temperature. After ensuring that there was no undissolved residue, the pH of the mixture was adjusted to about 7.0 with aqueous ammonia. Purified water was added to adjust the final concentration of muconic acid and cysteamine to 10% by mass.

The compositions of Experimental examples 19 and 20 were used to verify the hair treatment effects on human healthy hair. A bundle of 30 to 40 human healthy hairs or bleached hairs (average length: 25 cm) was immersed for 3 minutes in a liquid (pH: 7) prepared by diluting the composition of each of the experimental examples and the comparative example in such a manner that the concentration of dicarboxylic acid and thiol was 0.5% by mass, and excess water was wiped off. The hair was wound around a 15-mm hot rod with no gaps and heated for 10 minutes in such a manner that the hair surface was brought to 60°C. The hair was allowed to stand for another minute to cool the hot rod and removed from the hot rod. The hair was immersed in an aqueous shampoo solution at 40°C for 10 seconds, then rinsed with running water at 40°C for 10 seconds, and then wiped with a towel to remove water. The curl formation rate was then calculated. Table 18 presents the evaluation results. Comparison of the effects of Experimental examples 19 and 20 revealed that the curl was more persistent in Experimental example 19. The results indicated that the complex prepared by mixing in the acidic region provided a higher hair treatment effect.

**[Table 18]**

| | Experimental Example 19 | Experimental Example 20 |
|---|---|---|
| Curl Formation Rate (%) | 32.1 | 16.2 |

### 13. Evaluation of Hair Treatment Composition of Present Invention (12)

As Experimental example 21, a hair treatment composition was prepared by mixing cysteamine and muconic acid in a mole ratio of 2:1. Among the three isomers of muconic acid, trans-trans muconic acid was used. First, 1.42 g of muconic acid was added to 10 g of purified water and dissolved by the addition of aqueous ammonia. The mixture was heated to 80°C. After ensuring the dissolution of muconic acid, 2.27 g of cysteamine hydrochloride was added. The mixture was heated to boiling (up to 100°C) while mixing. The boiling state (100°C) was maintained for 1 hour to allow muconic acid and cysteamine to react with each other. Heating was stopped, and then the mixture was allowed to stand to normal temperature. After ensuring that there was no undissolved residue, the pH of the mixture was adjusted to about 7.0 with aqueous ammonia. Purified water was added to adjust the final concentration of muconic acid and cysteamine to 10% by mass.

In Experimental example 22, preparation was performed under the same conditions as in Experimental example 21, except that the reaction time between muconic acid and cysteamine was 6 hours. In Experimental example 23, preparation was performed under the same conditions as in Experimental example 21, except that cis-cis muconic acid was used instead of trans-trans muconic acid. In Experimental example 24, preparation was performed under the same conditions as in Experimental example 22, except that cis-cis muconic acid was used instead of trans-trans muconic acid.

In Experimental example 25, a hair treatment composition was prepared by mixing cysteamine and muconic acid in a mole ratio of 3:1 and was prepared under the same conditions as in Experimental example 24, except that 3.40 g of cysteamine hydrochloride was added to 1.42 g of cis-cis muconic acid. In Experimental example 26, a hair treatment composition was prepared by mixing cysteamine and muconic acid in a mole ratio of 1:1 and was prepared under the same conditions as in Experimental example 24, except that 1.13 g of cysteamine hydrochloride was added to 1.42 g of cis-cis muconic acid. In Experimental example 27, a hair treatment composition was prepared by mixing cysteamine and muconic acid in a mole ratio of 1:2 and was prepared under the same conditions as in Experimental example 24, except that 0.57 g of cysteamine hydrochloride was added to 1.42 g of cis-cis muconic acid.

The compositions of Experimental examples 21 to 27 were used to verify the hair treatment effects on human healthy hair by the same method as in "12.". Table 19 presents the preparation conditions and the curl formation rate of each experimental example. Good hair treatment effects were obtained in all experimental examples using any isomer of muconic acid. The use of trans-trans muconic acid indicated that highly effective complexes were prepared even with short reaction times. The highly effective complexes were prepared when cysteamine and muconic acid were mixed in a mole ratio of 1:1 to 3:1. However, in the case of using Experimental example 25 in which mixing was performed in a mole ratio of 3:1, a bad odor derived from thiol remained on the hair. The feel of the hair treated with Experimental example 25 was slightly inferior to Experimental example 24 or Experimental example 26.

**[Table 19]**

| Experimental Example | 2 1 | 22 | 2 3 | 24 | 25 | 2 6 | 27 |
|---|---|---|---|---|---|---|---|
| Mole Ratio (Cysteamine: Muconic Acid) | 2:1 | 2:1 | 2:1 | 2:1 | 3:1 | 1:1 | 1:2 |
| Structural Isomers of Muconic Acid | trans, trans | trans, trans | cis, cis | cis, cis | cis, cis | cis, cis | cis, cis |
| Reaction Time (Hour) | 1 | 6 | 1 | 6 | 6 | 6 | 6 |
| Curl Formation Rate (%) | 25.7 | 42.3 | 4.6 | 43.4 | 43.8 | 39.3 | 14.3 |

## Claims

1. A hair treatment composition, comprising:
as an active ingredient, a complex composed of a dicarboxylic acid and a thiol,
wherein the dicarboxylic acid is one or more selected from the group consisting of dicarboxylic acids each having 6 to 10 carbon atoms and cosmetically acceptable salts thereof, and
the thiol is one or more selected from the group consisting of aminothiols and cosmetically acceptable salts thereof.

2. The hair treatment composition according to Claim 1, wherein the dicarboxylic acid is one or more selected from the group consisting of adipic acid, pimelic acid, suberic acid, azelaic acid, hexenedioic acid, heptenedioic acid, octenedioic acid, muconic acid, and cosmetically acceptable salts thereof.

3. The hair treatment composition according to Claim 1, wherein the thiol is one or more selected from the group consisting of cysteamine, cysteine, amidinocysteine, aminothiophenol, mercaptopyridine, homocysteine, aminoundecanethiol, and cosmetically acceptable salts thereof.

4. The hair treatment composition according to Claim 1, wherein the dicarboxylic acid is one or more selected from the group consisting of adipic acid, octenedioic acid, muconic acid, and cosmetically acceptable salts thereof.

5. The hair treatment composition according to Claim 1, wherein the dicarboxylic acid is muconic acid or a cosmetically acceptable salt thereof, and the thiol is cysteamine or a cosmetically acceptable salt thereof.

6. The hair treatment composition according to Claim 1, wherein the complex, as the active ingredient, is obtained by reacting the dicarboxylic acid with the thiol at a mole ratio of thiol/dicarboxylic acid = 0.01 to 100.

7. The hair treatment composition according to Claim 6, wherein the mole ratio is thiol/dicarboxylic acid = 1 or more.

8. The hair treatment composition according to Claim 1, wherein the complex is obtained by a mixing step of mixing the dicarboxylic acid with the thiol at a high temperature.

9. The hair treatment composition according to Claim 8, wherein the mixing step is a mixing step of mixing the dicarboxylic acid with the thiol at 60°C to 100°C.

10. The hair treatment composition according to Claim 1, wherein the hair treatment composition is produced by a production method including at least a mixing step of mixing the dicarboxylic acid with the thiol at a high temperature.

11. The hair treatment composition according to Claim 10, wherein the mixing step is a mixing step of mixing the dicarboxylic acid with the thiol at 60°C to 100°C.

12. The hair treatment composition according to Claim 10, wherein in the mixing step, mixing is performed in an environment where an active ingredient other than the dicarboxylic acid, the thiol, a solvent therefor, and a pH adjuster is substantially absent.

13. The hair treatment composition according to any one of Claims 1 to 12, wherein the hair treatment composition contains substantially no active ingredient other than the dicarboxylic acid, the thiol, and the complex.

14. A hair straightening agent, a hair quality improver for use in dyeing or bleaching, a hair quality improver for use in a wave perm or a straight perm, a shampoo agent, a hair treatment agent, and/or a hair styling agent, comprising the hair treatment composition according to any one of Claims 1 to 12.

15. The hair quality improver for use in a wave perm or a straight perm according to Claim 14, wherein the hair quality improver is not a primary agent or a secondary agent for a wave perm or a straight perm.

16. A method for treating hair, comprising: at least an application step of applying the hair treatment composition according to any one of Claims 1 to 12 to hair; and
after the application step, a heating step of heating the hair.

17. The method for treating hair according to Claim 16, wherein the heating step is a step of heating the hair in such a manner that a surface temperature of the hair is 40°C to 180°C.

18. The method for treating hair according to Claim 16, wherein the heating step is a step of heating the hair in such a manner that the hair has a surface temperature of 40°C to 60°C.

19. The method for treating hair according to Claim 16, excluding a method for applying the hair treatment composition to hair as a primary agent or a secondary agent for a wave perm or a straight perm.

20. A method for treating hair according to Claim 16, wherein the method for treating hair is intended for one or more selected from the group consisting of hair straightening, improvements in hair quality during dyeing and bleaching, improvements in hair quality during a wave perm and a straight perm, shampooing, and hair treatment.
